# EUROPEAN PATENT SPECIFICATION

(11) **EP 4 179 072 B1**
(45) Date of publication and mention of the grant of the patent: **03.09.2025**
(21) Application number: 21740507.5
(22) Date of filing: 08.07.2021
(51) Int. Cl.: C12N 5/0775, A61K 35/28, C12N 5/00, C07K 14/435

(54) **PROCESS FOR THE MANUFACTURING OF PROTEIN-ASSOCIATED EXTRACELLULAR VESICLES**
VERFAHREN ZUR HERSTELLUNG VON PROTEINGEBUNDENEN EXTRAZELLULÄREN VESIKELN
PROCÉDÉ POUR LA FABRICATION DE VÉSICULES EXTRACELLULAIRES LIÉES À UNE PROTÉINE

(30) Priority: 09.07.2020 EP 20184901
(43) Date of publication of application: 17.05.2023
(73) Proprietor: EXO BIOLOGICS SA, 4000 Liège (BE)
(72) Inventor: JURGA, Marcin, 4000 Liège (BE)
(74) Representative: Brantsandpatents bv
(86) International application number: PCT/EP2021/068991
(87) International publication number: WO 2022/008654

(56) References cited:
- WO-A1-2012/125471
- WO-A1-2019/198077
- WO-A1-2020/257710
- WO-A1-2021/016368
- KAN YIN ET AL: "Exosomes from mesenchymal stem/stromal cells: a new therapeutic paradigm", BIOMARKER RESEARCH, BIOMED CENTRAL LTD, LONDON, UK, vol. 7, no. 1, 4 April 2019 (2019-04-04), pages 1 - 8, XP021271580, DOI: 10.1186/S40364-019-0159-X
- NATHALIE CHAPUT ET AL: "Exosomes: immune properties and potential clinical implementations", SEMINARS IN IMMUNOPATHOLOGY, SPRINGER, BERLIN, DE, vol. 33, no. 5, 21 December 2010 (2010-12-21), pages 419 - 440, XP019955366, ISSN: 1863-2300, DOI: 10.1007/S00281-010-0233-9
- XIN LUAN ET AL: "Engineering exosomes as refined biological nanoplatforms for drug delivery", ACTA PHARMACOLOGICA SINICA, VOL. 38, N.6, 10 April 2017 (2017-04-10), pages 754 - 763, XP055660177, Retrieved from the Internet <URL:https://www.ncbi.nlm.nih.gov/pmc/articles/PMC5520184/pdf/aps201712a.pdf> [retrieved on 20200121], DOI: 10.1038/aps.2017.12
- ANONYMOUS: "Advantages of calcium phosphate based excipients in pharmaceutical formulations development - Express Pharma", 26 November 2019 (2019-11-26), XP055753462, Retrieved from the Internet <URL:https://www.expresspharma.in/infrastructure/advantages-of-calcium-phosphate-based-excipients-in-pharmaceutical-formulations-development/> [retrieved on 20201124]
- NORDIN JOEL Z ET AL: "Tangential Flow Filtration with or Without Subsequent Bind-Elute Size Exclusion Chromatography for Purification of Extracellular Vesicles.", METHODS IN MOLECULAR BIOLOGY (CLIFTON, N.J.) 2019, vol. 1953, 2019, pages 287 - 299, XP009524296, ISSN: 1940-6029
- REKA AGNES HARASZTI ET AL: "Exosomes Produced from 3D Cultures of MSCs by Tangential Flow Filtration Show Higher Yield and Improved Activity", MOLECULAR THERAPY, vol. 26, no. 12, 1 December 2018 (2018-12-01), US, pages 2838 - 2847, XP055751842, ISSN: 1525-0016, DOI: 10.1016/j.ymthe.2018.09.015
- ZHENG ZHAO ET AL: "Microfluidic on-demand engineering of exosomes towards cancer immunotherapy", LAB ON A CHIP, vol. 19, no. 10, 1 January 2019 (2019-01-01), pages 1877 - 1886, XP055754271, ISSN: 1473-0197, DOI: 10.1039/C8LC01279B

## Description

### FIELD OF THE INVENTION

The present invention relates to a process for the manufacturing of extracellular vesicles (EVs) associated with proteins derived from mesenchymal stromal cells (MSCs). The current invention also relates to a composition comprising a therapeutically effective amount of EVs associated with proteins, and the use thereof.

### BACKGROUND

EVs are lipid bilayer-delimited particles that are naturally released from a cell and, unlike a cell, cannot replicate. EVs range in diameter from near the size of the smallest physically possible unilamellar liposome (around 20-30 nanometers) to as large as 10 microns or more, although the vast majority of EVs are smaller than 200 nm. They carry a cargo of proteins, nucleic acids, lipids, metabolites, and even organelles from the parent cell. Most cells that have been studied to date are thought to release EVs, including some bacterial, fungal, and plant cells that are surrounded by cell walls. A wide variety of EV subtypes have been proposed, defined variously by size, biogenesis pathway, cargo, cellular source, and function, leading to a historically heterogenous nomenclature including terms like exosomes, microvesicles and ectosomes.

EVs could be used for therapeutic purposes, such as delivering nucleic acids or other cargos to diseased tissue and cells. This growing interest was paralleled by formation of companies and funding programs focused on development of EVs as biomarkers or therapies of disease, the founding of an International Society for Extracellular Vesicles (ISEV), and establishment of a scientific journal devoted to the field, the Journal of Extracellular Vesicles.

With the growing interest in EVs for therapeutic use, also the need for clinical-grade EVs has risen. In order to be useful in the clinic, EVs need to be produced in a reproducible and controlled manner. Good Manufacturing Practice (GMP) for the manufacturing of compositions comprising EVs is crucial, in order to assure that various batches produced are uniform and of consistent quality. GMP is particularly difficult when dealing with cell-derived therapeutics. Several problems need to be solved before the clinical use of EVs can become widespread. It remains a challenge to develop platforms for the production, storage and handling of clinical grade EVs in a reliable and reproducibly quantifiable manner. MSC-derived EV-based pharmaceuticals and subsequent clinical trials demand the resolution of several technological, scientific, regulatory and mechanistic issues prior to bringing MSC-derived EV-based therapies to the clinic.

There is thus need for a protocol that enable the production of clinical-grade MSC-derived EVs in a stable and reproducible manner, under GMP conditions according to the Internal Conference on Harmonization (ICH) 2020 GMP quality guidelines. Moreover, there is a need for an EV-based product that has good stability and commercially attractive shelf life. In addition, there is a need to produce clinical-grade MSC derived EVs that have desired proteins bound on their membrane or surface. These proteins may enhance the functionality and the therapeutic value of the EVs.

EVs have been modified to express various therapeutic molecules (WO 2019/198 077) or have been loaded with siRNA, oligonucleotides or guide RNAs (Reka Agnes Haraszti et al., 2018). Loading can be done by incubation of donor cells with the cargo molecules or by sonication, extrusion freeze thaw cycles and electroporation of the EVs (Luan et a., 2017). WO2021/084274 A1 discloses a composition comprising EVs having a lipid-binding protein bound to the outer surface.

Methods for EVs purification have been reported but scalability is still a problem. Nordin Joel Z et al., 2019 discloses the use of tangential flow filtration (TFF) for purification of EVs as a putative method for large scale EVs production. The method employs the labor intensive bind-elute size exclusion chromatography. Zheng Zhao et al, 2019 proposes a method for production of surface-antigen engineered EVs by means of microfluidic cell culture chips. The latter are expensive and not readily available.

There remains a need in the art for production methods that allow efficient production of EVs associated with proteins, possibly for therapeutic use.

### SUMMARY OF THE INVENTION

The present invention and embodiments thereof serve to provide a solution to one or more of above-mentioned disadvantages. To this end, the present invention relates to a process for the manufacturing of EVs associated with proteins, derived from MSCs, according to claim 1. More particularly, said process comprises the steps of:
- purifying EVs from a cell medium comprising MSCs, wherein said purifying occurs via at least one filtration step of said medium;
- a concentration step of the filtrate of said at least one filtration step, wherein said EVs are concentrated by means of tangential flow filtration in a TFF device; and
- wherein during said TFF step the EVs are associated with one or more exogenous proteins inside of said TFF device, or in a vessel fluidly connected to said TFF device to which said EVs are transferred from said TFF.

In a second aspect, the present disclosure relates to a composition comprising a therapeutically effective amount of EVs associated with proteins. In a final aspect, the present disclosure relates to the use of a composition. Embodiments of the process are shown in claims 2-9.

### DESCRIPTION OF FIGURES

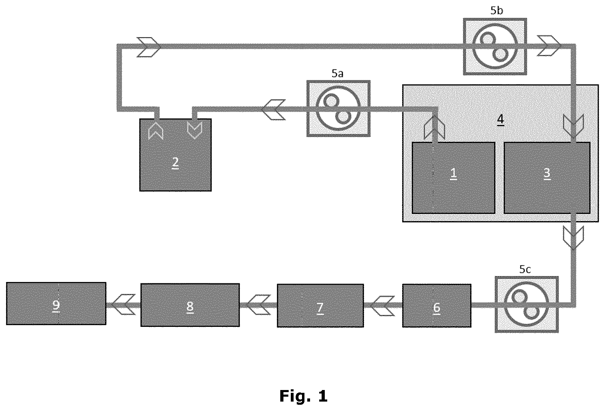

**Figure 1** shows an embodiment of a MSC expansion unit and EV processing unit that can be used to produce EVs of the current invention.

### DEFINITIONS

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g*., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

For the purpose of the current invention, the term "extracellular vesicles" or "EVs" is to be understood as micro or nano-meter sized particles secreted by different types of cells in vivo and in vitro including proteins associated with said EVs. EVs comprise proteins, growth factors, miRNA and other molecules encapsulated in a lipid sphere. EVs can be classified according to their size and intracellular origin. Exosomes are a subgroup of EVs that are typically in a size range of 0.1 micron or smaller. Exosomes are derived from multivesicular bodies, a late endosomal compartment, which are secreted via the fusion of multivesicular bodies with the plasma membrane. Another type of EV is the shedding vesicles (also known as microvesicles) which are a heterogenous population of membrane vesicles up to 1 micron in size directly released from the cell membrane through the disruption of the cortical cytoskeleton. All types of vesicles secreted by cells are defined in general as EVs.

The term "associated with EV(s)" in relation to substances means that said substance is either a) attached or bound to the surface layer of the EVs (by any type of binding, such as covalent or non-covalent binding) preferably by means of a non-covalent bond; and/or b) attached or bound within the surface layer of said EVs; and/or c) is internalized within said EVs.

Said substances associated with EVs may be any type of substances, such as, but not limited to molecules including amino acids, proteins, peptides, nucleic acids such as RNA and DNA (e.g. noncoding RNA, miRNA, mRNA), sugars, carbohydrates, fats, vitamins, growth factors, pro-angiogenic molecules, cardioprotective enzymes, antibodies, anti-inflammatory molecules, anti-fibrotic molecules, anti-oxidative molecules, pro-neurogenic molecules and anti-viral molecules; and ions such as metal ions or calcium ions.

The term "cell medium" or "cell culture medium" or "medium" refers to an aqueous solution of nutrients and other components of defined composition, which can be used for maintenance or growth of cells.

The term "pharmaceutically acceptable carrier" as used herein, refers to a carrier or a diluent that does not cause significant irritation to a subject and does not abrogate the biological activity and properties of the administered composition. As an example, the pharmaceutically acceptable carrier may act as a stabilizer and/or as an adjuvant. Examples, without limitations, of carriers are propylene glycol, saline, emulsions and mixtures of organic solvents with water.

The term "sufficient amount" means an amount sufficient to produce a desired and measurable effect, e.g., an amount sufficient to alter a protein expression profile.

The term "therapeutically effective amount" is an amount that is effective to ameliorate a symptom of a disease. A therapeutically effective amount can be a "prophylactically effective amount" as prophylaxis can be considered therapy.

The term "treatment" refers to both therapeutic treatment and prophylactic or preventative measures, wherein the objective is to prevent or slow down (lessen) the targeted pathologic condition or disorder. Those in need of treatment include those already with the disorder as well as those prone to have the disorder or those in whom the disorder is to be prevented.

The term " composition" refers to compositions at any stage of the manufacturing process, including the final pharmaceutically acceptable product and any in-process intermediates thereof.

The term "treating" refers to reversing, preventing, alleviating or inhibiting the progress of a disease, disorder or condition, or one or more symptoms of a disease, disorder or condition. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms of a disease, disorder or condition. As used herein, "treating" may also refer to reducing the severity of a disease, disorder or condition or symptoms of such disease, disorder or condition prior to affliction with the disease, disorder or condition. Such prevention or reduction of the severity of a disease, disorder or condition prior to affliction relates to the administration of the composition of the present technology, as described herein, to a subject that is not at the time of administration afflicted with the disease, disorder or condition. As used herein "treating" may also further refer to preventing the recurrence of a disease, disorder or condition or of one or more symptoms of such disease, disorder or condition. The terms "therapy," "treatment," and "therapeutically," as used herein, refer to the act of treating as defined above.

The term "fibrosis" as used herein refers to the formation of excess fibrous connective tissue in an organ or tissue in a reparative or reactive process.

For the purpose of the current invention, the term "mesenchymal stromal cells" or "MSCs" is to be understood as stromal adherent cells able to differentiate into various cell types. Sources of MSCs are bone marrow, cord cells, adipose tissue, amniotic fluid, mammary glands, blood.

### DETAILED DESCRIPTION OF THE INVENTION

The present disclosure concerns a process for the manufacturing of EVs associated with proteins, a composition comprising EVs associated with proteins and a use thereof. The passages of the disclosure that go beyond the scope of protection defined by the claims are not part of the invention.

In a first aspect, the present invention relates to a process for the manufacturing of EVs associated with proteins derived from MSCs, said process comprises the steps of:
- purifying EVs from a cell medium comprising MSCs, wherein said purifying occurs via at least one filtration step of said medium; followed by
- a concentration step of the filtrate of said at least one filtration step, wherein said EVs are concentrated by means of tangential flow filtration in a TFF device; and
- wherein during said TFF step the EVs are bound or associated with one or more exogenous proteins inside of said TFF device, or in a vessel fluidly connected to said TFF device to which said EVs are transferred from said TFF.

"Binding" and "binding to" in the context of the present invention may refer to any type of binding or attachment, either covalent or non-covalent, or any other type of binding or attachment known in the art.

The MSCs used in the process of the present invention are allowed to grow and expand in a concentrated cell medium. Once the MSCs have reached a desired concentration, a diluted cell medium comprising EVs, referred to as cell supernatant, from which EVs will be harvested, is collected for further processing. In an embodiment, the supernatant is collected when the MSCs reach a minimal concentration of at least 40x10⁶ cells/l. Cell concentration and viability may be defined by means of cell counting, such as for instance by means of a hemacytometer such as Bürker Counting Chamber and trypan blue staining.

To purify EVs from the supernatant, the cell medium in which the MSCs are maintained (also named the cell supernatant) is filtered, thereby removing contaminants present in said cell medium. The latter will contribute to the purity and stability of the end-product.

"Stability" of a product, such as EVs, in present context refers to the capability of a particular formulation or product in a specific environment, such as a container or closed system, to remain within a predefined range of values of parameters of physical, chemical, microbiological, toxicological and functional specifications or characteristics, for a given period of time. Non-limiting examples of such parameters are particle number, particle size, leakage of internal components and activity.

This purifying occurs via at least one filtration step of said medium. In an embodiment, said at least one filtration step is dead-end filtration.

In a preferred embodiment, said filtration comprises at least two steps of filtration. Also then, in an embodiment, at least one filtration step is dead-end filtration. In a further embodiment, said both filtration steps are performed by means dead-end filtration. Preferably, at least one of the filtration steps is also used as a product sterilization means, in order to be compliant with GMP regulations as defined above. Sequential dead-end filtration was in some cases found necessary to sufficiently remove impurities from the supernatant. In some cases, it was found that single filtration often resulted in blockage of the filter used and reduced the purity and quantity of the end product.

In a first filtration step, cell supernatant is filtered through a dead-end filter. In a further preferred embodiment, said filtration occurs by means of bringing said supernatant over a filter with mesh size of between 1 and 5 micron, more preferably between 1 and 3 micron. In an embodiment, said first filtration step is performed by means of dead-end filtration. In a further embodiment, this is preferably in a closed system with a peristaltic pump providing constant flow through the filter, even more preferably at about 100 ml/min.

In an embodiment, the filtrate of the first filtration will be brought over a second filter, this time a filter with a pore size that is smaller than the pore size used in the first filtration step. Preferably, this second filtration step is a sterilization step, in order to render the final product compliant with GMP regulations as defined above. In a more preferred embodiment, said filter has a mesh size below 1 micron, more preferably between 0.05 and 1 micron, even more preferably between 0.1 and 0.5 micron, most preferably between 0.1 and 0.22 micron. In an embodiment, said second filtration step is performed by means of dead-end filtration. In a further embodiment, this is preferably in a closed system interconnected with the first step of filtration. In another of further embodiment, this is with a peristaltic pump providing constant flow through the filter, even more preferably at about 100 ml/min.

The filtrate of said one or more filtration steps will comprise the EVs prepared according to the current invention.

In a final step, said EVs will be washed and concentrated. Washing and concentration may occur by conventional means known in the art such as membrane filtration, either micro- or ultra-filtration. Concentration is a process that involves removing fluid from a solution while retaining the solute molecules. Membrane filtration is a separation technique widely used in the life science laboratory. Depending on membrane porosity, it can be classified as a microfiltration or ultrafiltration process. Microfiltration membranes, with pore sizes typically between 0.1 µm and 10 µm, are generally used for clarification, sterilization, and removal of microparticulates or for cell harvesting. Ultrafiltration membranes, with much smaller pore sizes between 0.001 and 0.1 µm, are used for concentrating and desalting dissolved molecules (proteins, peptides, nucleic acids, carbohydrates, and other biomolecules), exchanging buffers, and gross fractionation. Ultrafiltration membranes are typically classified by molecular weight cutoff (MWCO) or nominal molecular weight cutoff (NMWCO) rather than pore size.

There are two main membrane filtration modes which can use either microfiltration or ultrafiltration membranes: 1) Direct Flow Filtration (DFF), also known as "dead-end" filtration, applies the feed stream perpendicular to the membrane face and attempts to pass 100% of the fluid through the membrane, and 2) Tangential Flow Filtration (TFF), also known as Crossflow Filtration, where the feed stream passes parallel to the membrane face as one portion passes through the membrane (permeate) while the remainder (retentate) is recirculated back to the feed reservoir.

By preference said washing and concentration will be performed by means of tangential filtration step (TFF). Tangential Flow Filtration (TFF) or Crossflow Filtration is a process where the feed stream flows parallel to the membrane face. Applied pressure causes one portion of the flow stream to pass through a membrane (permeate) while the remainder (retentate) is recirculated back to the feed reservoir.

In an embodiment, the filtrate of the at least one filtration step comprising said EVs derived from MSCs will be used in a TFF concentration step in a TFF device.

In a further preferred embodiment, the TFF will have a (molecular weight) cut-off range (MWCO) of 100 kDa. MWCO is defined as the minimum molecular weight of a solute that is 90 % retained by the membrane. As such, TFF with a cut-off range of 100 kDa will remove the majority if not all particles and components with a molecular weight lower than 100 kDa into the TFF permeate. Consequently, said final composition, which remains in the retentate, does not comprise free (that is: not EV-associated) constituents, components or substances with a molecular weight below 100 kDa. Said constituents, components or substances can be any particle such as a protein or a peptide that is normally present or part of said cell medium.

In an embodiment, during said TFF step the EVs are bound or associated with one or more exogenous proteins inside said TFF device or said vessel which is fluidly connected to said TFF device to which said EVs are transferred from said TFF. In an embodiment, said proteins are supplemented to said TFF device or said connected vessel during concentrating of said EVs. In a further embodiment, said supplementing of proteins to said TFF device or connected vessel can, as non-limiting examples, be by infusion or injection into the TFF device, preferably is a closed system.

Said proteins will bind or associate with the EVs in the TFF or vessel connected to the TFF. Importantly, said vessel should be fluidly connected to the TFF, in a closed system. Said vessel can be any vessel known in the art which can be connected to the TFF device and in which the TFF retentate comprising the EVs can be combined with the required proteins. In a non-limiting example, said vessel can be a bag or pouch. Subsequently, the combined product with retentate and proteins will be transferred back to the TFF.

Unbound or unassociated proteins will be washed away during one or more washing steps which are performed preferably in the TFF. Washing steps can be performed using one or more washing buffers, such as a saline buffer solution. Washing buffers should be compatible with the EVs and proteins, in a way that the buffers cannot comprise any toxic substances.

In an embodiment, said proteins are selected from the group of annexins, thioredoxins or lactadherin. In a preferred embodiment, said protein is Annexin V, Trx or Mfge8, preferably recombinant Annexin V, Trx or Mfge8.

Annexins are calcium dependent phospholipid binding proteins for which binding to EVs increases anti-inflammatory properties of EVs. Some annexins, such as Annexin V, are associated with pro-inflammatory activity. However it was shown that association of Annexin V to EVs increased anti-inflammatory activity of EVs. Annexin V has a molecular weight of about 37 kDa.

Thioredoxins are small redox proteins known to be present in all organisms. Thioredoxin 1 (Trx) has a strong anti-oxidative activity, has anti-inflammatory and anti-fibrotic effects. In addition, binding to EVs increases the anti-inflammatory effects of EVs.

Lactadherin, also referred to as milk fat globule-EGF factor 8 protein (Mfge8), is a secreted protein found in vertebrates, including mammals as well as birds. It comprises a phophatidylserine binding domain and an Arg-Gly-Asp binding sequence. Mfge8 binds in a calcium independent manner.

In a further embodiment, calcium is supplemented to said TFF device or in said fluidly connected vessel during concentrating of said EVs. Preferably a calcium concentration of between about 1 and about 10 mM is supplemented. In an example, this is between 1 and 9 mM, in another example between 1 and 8 mM, in another example between 1 and 7 mM, in another example between 1 and 6 mM, in another example between 1 and 5 mM, in another example between 1 and 4 mM, in another example between 1 and 3 mM. Preferably this is between 1.5 and 3 mM, preferably between 1.5 and 2.5 mM. Most preferably about 2 mM calcium is supplemented to said TFF device during concentrating of said EVs.

Calcium in human blood is in general maintained in a fairly narrow range from about 2.2 to about 2.7 mM. If said EVs are to be administered to a (human) patient (see further), calcium ranges are to be maintained preferably below 10 mM in the final administered product.

Infusion of calcium in the TFF device or said fluidly connected vessel aids the binding of calcium-dependent proteins to EVs, such as but not limited to Annexins and Thioredoxins. For proteins binding to or associating with EVs in a calcium-independent manner, such as Mfge8, infusion of calcium is not required.

In a further embodiment, said calcium and said proteins are mixed in said TFF or fluidly connected vessel and incubated with said EVs residing in said TFF device or vessel.

In a further embodiment, said protein-bound EVs/EVs associated with proteins are washed, re-concentrated and subsequently collected outside TFF device.

As already indicated before, proteins which are not bound to or associated with the EVs will be removed during one or more washing steps which are preferably performed in the TFF as well. The retentate will comprises the EVs. One or more washing steps can be performed using washing buffers, such as but not limited to a saline buffer solution. Washing buffers should be compatible with the EVs and proteins associated with them, in a sense that the EVs and proteins associated with them remain viable and retain their properties.

When unbound or unassociated proteins have been sufficiently washed away, the protein-bound EVs/EVs associated with said proteins are concentrated, preferably by means of circulation through the TFF device. The concentrated final product comprising EVs is subsequently collected outside the device, preferably in a collection vessel, such as but not limited to a collection bag or cryotubes.

Storage of the final product may occur below 10°C, preferably at or below 4°C or more preferably by cryopreservation wherein said composition is frozen at a temperature of between -20°C and -196°C, preferably between -40°C and -196°C, more preferably between -80°C and -196°C. Said freezing procedure is preferably a snap-freezing or vitrification procedure, ensuring that the product remains viable during the freezing process and after thawing. Another freezing procedure can be controlled rate freezing, preferably with compensation of the exothermic reaction at the crystallization point for better product stability. For the latter procedure, a controlled rate freezer can be used.

In an embodiment, the medium in which MSCs are cultured and expanded before dilution and the start of EV harvest, is a cell medium comprising purified human serum albumin.

Albumin in the end-product aids in the stability and functionality of said EVs. As previously defined, "stability" of a product, such as EVs, in present context refers to the capability of a particular formulation or product in a specific environment, such as a container or closed system, to remain within a predefined range of values of parameters of physical, chemical, microbiological, toxicological and functional specifications or characteristics, for a given period of time. Non-limiting examples of such parameters are particle number, particle size, leakage of internal components and activity.

As it aids in the stability and functionality of EVs, albumin should preferably be present in said cell medium for culturing and expansion. In an embodiment, said medium therefore comprises human albumin, either recombinant or purified albumin, e.g. purified from human plasma. In a further embodiment, said albumin is present in said medium at a concentration of between 1 g/l and 5 g/l. The latter concentration was shown to be particularly useful in obtaining an active and stable end product.

It is also disclosed a composition comprising a therapeutically effective amount of protein-bound EVs/EVs associated with proteins, and optionally calcium. Said composition is obtainable by the process described above. In a preferred embodiment, said EVs will have a size below 1 µm.

In a preferred embodiment, the EVs of the composition have a size of about below 750 nm, preferably below 500 nm, preferably below 400 nm, preferably below 300 nm. In another or further preferred embodiment, the EVs of the composition have a size of at least 5 nm, more preferably at least 10 nm, more preferably at least 25 nm, more preferably at least 50 nm. In another or further preferred embodiment, the EVs of the composition have a size of between 25 and 500 nm, preferably between 25 and 400 nm, more preferably between about 50 and about 300 nm.

Optical techniques are routinely used to size and count extracellular vesicles (EV). In an embodiment of the current disclosure, the particle size of said EVs is measured by Nanoparticle Tracking Analysis (NTA), which is a preferred method for quantification and sizing of nanoparticles suspended in liquid buffers. In another of further embodiment, the particle size is measured using Tunable Resistive Pulse 25 Sensing (TRPS) which is used as a reference method to NTA. In another or further embodiment, the particle size is measured using High-Resolution Flow Cytometry.

An effective amount is that amount of an agent that alone stimulates the desired outcome. The absolute amount will depend upon a variety of factors, including the material selected for administration, whether the administration is in single or multiple doses, and individual patient parameters including age, physical condition, size, weight, and the stage of the disease. These factors are well known to those of ordinary skill in the art and can be addressed with no more than routine experimentation. Dosage may also differ depending on the subject to which said composition is administered.

In an embodiment of the composition, said composition may further comprises calcium, preferably the concentration of calcium in said composition is between about 1 and about 10 mM. In an embodiment, said calcium concentration is between 1 and 9 mM, or between 1 and 8 mM, or between 1 and 7 mM, or between 1 and 6 mM, or between 1 and 5 mM, or between 1 and 4 mM, or between 1 and 3 mM. Preferably this is between 1.5 and 3 mM, preferably between 1.5 and 2.5 mM. Most preferably the calcium concentration in the compositon is about 2 mM.

In an embodiment, said calcium (at least partly) binds to or associates with the EVs. Less than 1 mM calcium will lead to a reduced binding of calcium-dependent binding proteins. As calcium in human blood is in general maintained in a fairly narrow range from about 2.2 to about 2.7 mM, administration of a composition to a human patient, for which the composition comprises more than 10 mM calcium, is to be avoided.

In an embodiment, the composition according to the current disclosure will further comprise human albumin concentration of between 10 and 30 g/l, more preferably at a concentration of between 10 and 20 g/l, more preferably between 15 and 20 g/l.

The source of said albumin is the cell medium for MSCs. While albumin might thus theoretically be considered a contaminant of the production process as it is not produced by the MSCs, it was surprisingly found that the presence of albumin is in fact needed to ensure the stability and activity of the end-product and thus the composition of the current disclosure. Albumin at the required concentration acts as a drug stabilizer and enhancer of activity. It was found that forced removal of albumin reduces the activity of the product (Hyungtaek Jeon et al., 2020).

Said albumin concentration may be measured by means of colorimetric methods or ELISA by means of an anti-albumin antibody. In an embodiment, said colorimetric measurement is a bromocresol green assay.

In an embodiment, at least 90% of said albumin present in said composition is associated with said EVs in said composition.

Human albumin has a MW of around 66 kDa. Albumin not associated with EVs will therefore be removed by TFF treatment, the remaining albumin in the composition being associated with the EVs. In a further preferred embodiment, at least 93%, more preferably 94%, more preferably 95%, more preferably 96%, more preferably 97%, more preferably 98%, more preferably 99% of the albumin in said composition is associated with EV.

In an embodiment, a composition according to the current disclosure comprises human albumin and calcium, preferably the composition comprises between about 25 and 750 mg albumin per mg calcium, more preferably between about 25 and 500 mg albumin per mg calcium, more preferably between about 50 and 250 mg albumin per mg calcium, more preferably between about 50 and 500 mg albumin per mg calcium, more preferably between about 75 and 400 mg albumin per mg calcium, more preferably between about 100 and 350 mg albumin per mg calcium, more preferably between about 125 and 300 mg albumin per mg calcium, more preferably between about 150 and 275 mg albumin per mg calcium, most preferably between about 175 and 250 mg albumin per mg calcium.

In an embodiment of the composition, said proteins associated with EVs are selected from annexins, thioredoxins and lactadherin, preferably said proteins are recombinant proteins. In a further embodiment of the composition, said protein is Annexin V, Trx or Mfge8, preferably recombinant Annexin V, Trx or Mfge8.

As indicated before, annexins are calcium dependent phospholipid binding proteins for which binding to EVs increases anti-inflammatory properties of EVs. Some annexins, such as Annexin V, are associated with pro-inflammatory activity. However it was shown that association of Annexin V to EVs increased anti-inflammatory activity of EVs. Thioredoxins are small redox proteins known to be present in all organisms. Thioredoxin 1 (Trx) has a strong anti-oxidative activity, has anti-inflammatory and anti-fibrotic effects. In addition, binding to EVs increases the anti-inflammatory effects of EVs. Lactadherin, also referred to as milk fat globule-EGF factor 8 protein (Mfge8), is a secreted protein found in vertebrates, including mammals as well as birds. It comprises a phophatidylserine binding domain and an Arg-Gly-Asp binding sequence. Mfge8 binds in a calcium independent manner.

In an embodiment, the composition comprises EVs associated with Annexin V, preferably recombinant Annexin V, and further comprises calcium. In an embodiment, the ratio between EV-associated Annexin V and albumin is preferably between 1:90000 and 1:3000000.

In an embodiment of the disclosure the composition may further comprise one or more secondary therapeutic agents. As used herein, a therapeutic agent refers to any agent which can be used in the prevention, treatment and/or management of a disease such as those discussed herein. Such agents may be intra-vesicular (i.e., contained within the body of the EVs) or may be associated with the EVs. Suitable therapeutic agents will be known to the skilled person and might include noncoding RNA, miRNA, mRNA, growth factors, pro-angiogenic molecules, cardioprotective enzymes, antibodies, anti-inflammatory molecules, anti-fibrotic molecules, anti-oxidative molecules, pro-neurogenic molecules, anti-viral molecules, etc. In some embodiments, the isolated EVs are used together with a secondary agent. In some embodiments, the secondary agent is a steroid, an antioxidant, or inhaled nitric oxide. In some embodiments, the steroid is a corticosteroid. In some embodiments, the corticosteroid is methylprednisolone or dexamethasone. In some embodiments, the antioxidant is superoxide dismutase.

Certain secondary therapeutic agents used in the treatment or management of certain lung diseases including but not limited to pulmonary hypertension include oxygen, anticoagulants such as warfarin (Coumadin); diuretics such as furosemide (Lasix^{®}) or spironalactone (Aldactone^{®}); calcium channel blockers; potassium such as K-dur^{®}; inotropic agents such as digoxin; vasodilators such as nifedipine (Procardia^{®}) or diltiazem (Cardizem^{®}); endothelin receptor antagonists such as bosentan (Tracleer^{®}) and ambrisentan (Letairis^{®}); prostacyclin analogues such as epoprostenol (Flolan^{®}), treprostinil sodium (Remodulin^{®}, Tyvaso^{®}), and iloprost (Ventavis^{®}); and PDE-5 inhibitors such as sildenafil (Revatio^{®}) and tadalafil (Adcirca^{®}).

Said composition will preferably be formulated as a liquid. Storage may be done by means of vials, IV bags, ampoules, cartridges, inhalers such as liquid inhalers, nebulizers or powder inhalers, and prefilled syringes. Said liquid formulation may, apart from the EVs as active principle or drug product further comprise a variety of compounds to ensure a stable active medication following storage. These include solubilizers, stabilizers, buffers, tonicity modifiers, bulking agents, viscosity enhancers/reducers, surfactants, chelating agents, and adjuvants.

In another embodiment, said composition may be lyophilized or freeze dried. Said lyophilized formulation may be stored in vials, cartridges, inhalers such as liquid inhalers, nebulizers or powder inhalers, dual chamber syringes, and prefilled mixing systems. Before administration, a lyophilized composition is reconstituted as a liquid before being administered. This may be done by combining a liquid diluent with the freeze-dried powder, mixing, then injecting. Reconstitution usually requires a reconstitution and delivery system to ensure that the drug is correctly mixed and administered.

In a preferred embodiment, said composition is aqueous. In one embodiment of the disclosure the EVs will be formulated as a composition which further comprises a pharmaceutically acceptable carrier. The pharmaceutically acceptable carrier or diluent is chosen wherein the EVs of the disclosure remain viable and retain their properties. A pharmaceutically acceptable carrier is a pharmaceutically acceptable material, composition or vehicle, such as a liquid or solid filler, diluent, excipient, solvent or encapsulating material, involved in carrying or transporting a prophylactically or therapeutically active agent. Each carrier must be "acceptable" in the sense of being compatible with the other ingredients of the formulation and not injurious to the subject. Some examples of materials which can serve as pharmaceutically acceptable carriers include sugars, such as lactose, glucose and sucrose; glycols, such as propylene glycol; polyols, such as glycerin, sorbitol, mannitol and polyethylene glycol; esters, such as ethyl oleate and ethyl laurate; buffering agents, such as magnesium hydroxide, magnesium stearate and aluminum hydroxide; pyrogen-free water; isotonic saline; Ringer's solution; ethyl alcohol; phosphate buffer solutions; and other nontoxic compatible substances employed in pharmaceutical formulations.

Storage of said composition may occur at 4°C or more preferably by cryopreservation wherein said composition is frozen at a temperature of between -20°C and -196°C, preferably between -40°C and -196°C, more preferably between -80°C and -196°C. Said freezing procedure is preferably a snap-freezing or vitrification procedure, ensuring that the product remains viable during the freezing process and after thawing. Another freezing procedure can be controlled rate freezing, preferably with compensation of the exothermic reaction at the crystallization point for better product stability. For the latter procedure, a controlled rate freezer can be used.

In an embodiment, said composition will be suitable and/or will be formulated for administration to a patient via injection, intravenous administration, subcutaneous administration, intramuscular administration, cutaneous or transdermal administration, inhalation, intratracheal infusion, oral infusion, systemic infusion, intranasal infusion. Said composition may also be formulated to be used externally, alone or in combination with hydrogels, polymers or polymer medical devices for slow release of EVs.

In some embodiments, the composition is suited for intravenous administration. In some embodiments, the composition is suited for administration to lungs or trachea of a subject. In some embodiments, the composition is formulated for administration by inhalation. In some embodiments, the composition is formulated for administration as an aerosol. In some embodiments, the EVs are administered using a nebulizer. In some embodiments, the isolated EVs are administered using an intratracheal tube.

In some embodiments, the EVs are administered or formulated with a surfactant, preferably a pulmonary surfactant. This surfactant preferably is chosen in such way as not to affect the stability of the composition. In some embodiments, the pulmonary surfactant is isolated naturally occurring surfactant. In some embodiments, the pulmonary surfactant is derived from bovine lung or porcine lung. In some embodiments, the pulmonary surfactant is a synthetic surfactant. A pulmonary surfactant is a lipoprotein mixture useful in keeping lung airways open (e.g., by preventing adhesion of alveolar walls to each other). Pulmonary surfactants may be comprised of phospholipids such as dipalmitoylphosphatidylcholine (DPPC), - phosphotidylcholine (PC), phosphotidylglycerol (PG); cholesterol; and proteins such as SP-A, B, C and D. Pulmonary surfactants may be derived from naturally occurring sources such as bovine or porcine lung tissue. Examples include Alveofact^{™} (from cow lung lavage), Curosurf^{™} (from minced pig lung), Infasurf^{™} (from calf lung lavage), and Survanta^{™} (from minced cow lung, with additional components including DPPC, palmitic acid, and tripalmitin). Pulmonary surfactants may also be synthetic. Examples include Exosurf^{™} (comprised of DPPC with hexadecanol and tyloxapol), Pumactant^{™} or Artificial Lung Expanding Compound (ALEC) (comprised of DPPC and PG), KL-4 (comprised of DPPC, palmitoyl-oleoyl phosphatidylglyercol, palmitic acid, and synthetic peptide that mimics SP-B), Venticute^{™} (comprised of DPPC, PG, palmitic acid, and recombinant SP-C). Pulmonary surfactants may be obtained from commercial suppliers.

The disclosure also encompasses a packaged and labelled pharmaceutical product. This article of manufacture or kit includes the appropriate unit dosage form in an appropriate vessel or container such as a glass vial or plastic ampoule or other container that is hermetically sealed. The unit dosage form should be suitable for pulmonary delivery for example by aerosol. Preferably, the article of manufacture or kit further comprises instructions on how to use including how to administer the pharmaceutical product. The instructions may further contain informational material that advises a medical practitioner, technician or subject on how to appropriately prevent or treat the disease or disorder in question. In other words, the article of manufacture includes instructions indicating or suggesting a dosing regimen for use including but not limited to actual doses, monitoring procedures, and other monitoring information.

As with any pharmaceutical product, the packaging material and container are designed to protect the stability of the product during storage and shipment, and may contain desiccants to ensure stability.

The kits may include EVs in sterile aqueous suspensions that may be used directly or may be diluted with normal saline for intravenous injection or use in a nebulizer, or dilution or combination with surfactant for intratracheal administration. The kits may therefore also contain the diluent solution or agent, such as saline or surfactant. The kit may also include a pulmonary delivery device such as a nebulizer or disposable components therefore such as the mouthpiece, nosepiece, or mask.

In a final aspect, the current disclosure relates to the use of the composition as described above. More specifically, said composition is suited for therapeutic or prophylactic use. It is contemplated the prevention and treatment of certain diseases. Preventing a disease means reducing the likelihood that the disease manifests itself and/or delaying the onset of the disease. Treating a disease means reducing or eliminating the symptoms of the disease. It is also contemplated the provision of a method for treating and/or preventing a disease in a subject.

The subjects are preferably human subjects, but certain aspects of the current disclosure may be performed on any subject likely to derive benefit therefrom, including human subjects, agricultural livestock (e.g., cows, pigs, etc.), prized animals (e.g., horses), companion animals (e.g., dogs, cats, etc.), and the like. In a preferred embodiment, said subject is a human, preferably a human patient, said patient may be an adult, an infant or a neonate.

In an embodiment, the composition according to any of the embodiments above is used in the prevention or treatment of lung disorders. In an embodiment, said lung disorder may be an inflammatory lung disease, lung vascular disease, or acute lung injury. More preferably, said inflammatory lung disease is pulmonary hypertension which is also referred to as pulmonary artery hypertension (PAH), asthma, bronchopulmonary dysplasia (BPD), allergy, idiopathic pulmonary fibrosis or pneumonia. Said inflammatory lung disease may be caused by an infection, such as a viral infection. In a preferred embodiment, said viral infection is influenza, SARS-CoV-1, MERS, or SARS-CoV-2. In another embodiment, said acute lung injury is linked with sepsis or is acute respiratory distress syndrome (ARDS).

These diseases also include lung vascular diseases which may not have an inflammatory component. Still other pulmonary conditions that may be treated include acute lung injury which may be linked with sepsis or with ventilation. An example of this latter condition is acute respiratory distress syndrome.

Pulmonary hypertension is a lung disease characterized by blood pressure in the pulmonary artery that is far above normal levels. Symptoms include shortness of breath, chest pain particularly during physical activity, weakness, fatigue, fainting, light headedness particularly during exercise, dizziness, abnormal heart sounds and murmurs, engorgement of the jugular vein, retention of fluid in the abdomen, legs and ankles, and bluish coloring in the nail bed.

Bronchopulmonary dysplasia is a condition that afflicts neonates who have been given oxygen or have been on ventilators, or neonates born prematurely particularly those born very prematurely (e.g., those born before 32 weeks of gestation). It is also referred to as neonatal chronic lung disease. Causes of BPD include mechanical injury for example as a result of ventilation, oxygen toxicity for example as a result of oxygen therapy, and infection. The disease may progress from non-inflammatory to inflammatory with time. Symptoms include bluish skin, chronic cough, rapid breathing, and shortness of breath. Subjects having BPD are more susceptible to infections such as respiratory syncytial virus infection. Subjects having BPD may develop pulmonary hypertension.

Acute respiratory distress syndrome (ARDS), also known as respiratory distress syndrome (RDS) or adult respiratory distress syndrome is a condition that arises as a result of injury to the lungs or acute illness. The injury to the lung may be a result of ventilation, trauma, burns, and/or aspiration. The acute illness may be infectious pneumonia or sepsis. It is considered a severe form of acute lung injury, and it is often fatal. It is characterized by lung inflammation, impaired gas exchange, and release of inflammatory mediators, hypoxemia, and multiple organ failure. ARDS can also be defined as the ratio of arterial partial oxygen tension (PaO2) as a fraction of inspired oxygen (FiO2) below 200 mmHg in the presence of bilateral infiltrates on the chest x-ray. A PaO2/FiO2 ratio less than 300 mmHg with bilateral infiltrates indicates acute lung injury, which is often a precursor to ARDS. Symptoms of ARDS include shortness of breath, tachypnea, and mental confusion due to low oxygen levels.

Idiopathic pulmonary fibrosis is characterized by scarring or thickening of the lungs without a known cause. It occurs most often in persons 50-70 years of age. Its symptoms include shortness of breath, regular cough (typically a dry cough), chest pain, and decreased activity level.

Prevention and/or treatment may involve in some instances use of the EVs alone or together with one or more secondary agents or active ingredients. Subjects may also be subjected to mechanical interventions such as ventilation with or without exogenous oxygen administration.

The subjects may be those that have a lung disease (or condition) amenable to treatment using the EVs of the disclosure, or they may be those that are at risk of developing such a disease (or condition). Such subjects include neonates and particularly neonates born at low gestational age. As used herein, a human neonate refers to an human from the time of birth to about 4 weeks of age. As used herein, a human infant refers to a human from about the age of 4 weeks of age to about 3 years of age. As used herein, low gestational age refers to birth (or delivery) that occurs before a normal gestational term for a given species. In humans, a full gestational term is about 40 weeks and may range from 37 weeks to more than 40 weeks. Low gestational age, in humans, akin to a premature birth is defined as birth that occurs before 37 weeks of gestation. It is therefore contemplated the prevention and/or treatment of subjects born before 37 weeks of gestation, including those born at even shorter gestational terms (e.g., before 36, before 35, before 34, before 33, before 32, before 31, before 30, before 29, before 28, before 27, before 26, or before 25 weeks of gestation). Typically such premature infants will be treated as neonates, however it is contemplated their treatment even beyond the neonate stage and into childhood and/or adulthood. Certain subjects may have a genetic predisposition to certain forms of lung disease such as for example pulmonary hypertension, and those subjects may also be treated.

With respect to neonates and particularly low gestation age neonatesit is contemplated the administration of EVs within 4 weeks, 3 weeks, 2 weeks, 1 week, 6 days, 5 days, 4 days, 3 days, 2 days, 1 day, 12 hours, 6 hours, 3 hours, or 1 hour of birth. In some important instances, the MSC exosomes are administered within 1 hour of birth.

The disclosure further contemplates administration of EVs even in the absence of symptoms indicative of a lung disease such as but not limited to BPD.

In an embodiment, said composition comprising EVs according to the current disclosure may be used in treatment of COVID-19, more specifically COVID-19 induced or acute pneumonia. COVID-19, a novel infectious disease caused by severe acute respiratory syndrome, attacking the human respiratory system and lung's epithelial tissue. It has been reported that a subpopulation of patients is at higher risk of developing more severe symptoms of COVID-19 disease. Major complications include pneumonia, acute respiratory distress syndrome (ARDS), multi-organ failure, septic shock, and death.

It has been shown that COVID-19, also referred to as SARS-CoV-2, is related to different mechanisms of lung infections that can progress to acute respiratory distress syndrome (ARDS) likely precipitated by a cytokine storm, multi-organ failure, septic shock, and blood clots. In comparison to bacterial pneumonia, which is a common lung infection where the entire lung or a section of the lungs' air sacks become inflamed and are filled with fluid, pus, and cellular debris and is mostly caused by viruses, fungi, or bacteria and often treated with antibiotics. In other cases, cardiovascular complications occur, elevated liver enzymes reflecting liver injury, neurologic manifestations. In children, if the infection progresses, it develops pediatric multisystem inflammatory syndrome, which has symptoms similar to Kawasaki disease, which could resulting in death. Based on the current data, children make up a small proportion of reported cases, with about 1% of cases being under 10 years and 4% aged 10-19 years.

Said composition comprising EVs provides a multi-target therapeutic effect with their main mode of action being the inhibition of an inflammatory process.

The EVs target multiple mechanisms of lung injury including hyper-inflammation and cytokine storm, fibrosis, oxidative stress caused by (mechanical) ventilation and epithelial cells apoptosis due to viral activity and inflammatory reaction.

In another embodiment, said composition comprising EVs is used as a supplementary treatment of COVID-19, more specifically COVID-19 induced or acute pneumonia.

In another embodiment, said composition according to any of the embodiments above is used in the prevention or treatment of inflammatory bowel diseases (IBD) such as Crohn's Disease or ulcerative colitis. IBD are a collection of diseases that result in an inflammation of the gastrointestinal (GI) tract, and the two most common IBD are ulcerative colitis (UC) and Crohn's disease (CD). UC is a disease that causes long-lasting inflammation and sores in the innermost lining of the colon and rectum. CD can develop anywhere in the digestive tract and can penetrate into the deep layers of the affected tissue. A symptom of CD is the development of perianal fistulas. The diseases are similar, however, in that both can cause abdominal pain, severe diarrhea, fatigue and weight loss.

It was shown that a composition according to the disclosure, comprising a therapeutically effective amount of EVs associated with Annexin V, preferably recombinant Annexin V, is particularly effective for use in the prevention or treatment of inflammatory bowel diseases, particularly in Crohn's Disease and ulcerative colitis, preferably in Crohn's Disease.

In case of administration of EVs in the context of a lung disease, the preferred way of administration will be intratracheal infusion or inhalation. In the context of neurology diseases, this will preferably be systemic infusion, intranasal infusion or inhalation. In the context of Crohn's disease fistulas, ulcers or cartilage repair, this will preferably be via local injection(s). In the case of treatment of wound healing, burns and/or ulcers, the EVs will preferably be administered at the external side of the body, preferably in combination with hydrogels or polymer medical devices for slow release of EVs.

The EVs of the disclosure are administered in effective amounts. An effective amount is that amount of an agent that alone stimulates the desired outcome. The absolute amount will depend upon a variety of factors, including the material selected for administration, whether the administration is in single or multiple doses, and individual patient parameters including age, physical condition, size, weight, and the stage of the disease. These factors are well known to those of ordinary skill in the art and can be addressed with no more than routine experimentation. Dosage may also differ depending on the subject to which said composition is administered.

In an embodiment, said EVs are administered at a dose of 10⁹ EVs/kg to 10¹² EVs/kg of said patient, or at a dose of 10¹⁰ EVs/kg to 10¹² EVs/kg of said patient. In a further embodiment, dosage ranges from 10⁹ EVs/kg to 10¹¹ EVs/kg in children (from the age of 0 months to 12 year). In teenagers and adults, said dose may range from 10⁹ EVs/kg to 10¹² EVs/kg in adults.

In an embodiment of the current disclosure, said EVs are administered at a dose of about 10¹⁰ to about 10¹² EVs per patient for the treatment of CD perianal fistulas.

The disclosure also contemplates repeated administration of EVs, including two, three, four, five or more administrations. In some instances, said EVs may be administered continuously. Repeated or continuous administration may occur over a period of several hours (e.g., 1-2, 1-3, 1-6, 1-12, 1-18, or 1-24 hours), several days (e.g., 1-2, 1-3, 1-4, 1-5, 1-6 days, or 1-7 days) or several weeks (e.g., 1-2 weeks, 1-3 weeks, or 1-4 weeks) depending on the severity of the condition being treated. If administration is repeated but not continuous, the time in between administrations may be hours (e.g., 4 hours, 6 hours, or 12 hours), days (e.g., 1 day, 2 days, 3 days, 4 days, 5 days, or 6 days), or weeks (e.g., 1 week, 2 weeks, 3 weeks, or 4 weeks). The time between administrations may be the same or they may differ. As an example, if the symptoms of the disease appear to be worsening the EVs may be administered more frequently, and then once the symptoms are stabilized or diminishing the EVs may be administered less frequently.

In some instances, repeated intravenous administration of low doses of EVs may occur. Accordingly, the disclosure contemplates repeated administration of low dosage forms of EVs as well as single administrations of high dosage forms of EVs. Low dosage forms may range from, without limitation, 10¹⁰ - 10¹¹ EVs per kilogram or per local infusion, while high dosage forms may range from, without limitation, 0¹¹ - 10¹² per kilogram or per local infusion. It will be understood that, depending on the severity of the disease, the health of the subject, and the route of administration, inter alia, the single or repeated administration of low or high dose EVs are contemplated.

The EVs may be administered by any route that effects delivery to the lungs or the gastrointestinal tract. Systemic administration routes such as intravenous bolus injection or continuous infusion are suitable. More direct routes such as intranasal administration, intratracheal administration (e.g., via intubation), and inhalation (e.g., via an aerosol through the mouth or nose) are also contemplated and in some instances may be more appropriate particularly where rapid action is necessary. As used herein, an aerosol is a suspension of liquid dispersed as small particles in a gas, and it includes a fine mist or a spray containing such particles. As used herein, aerosolization is the process of producing of an aerosol by transforming a liquid suspension into small particles or droplets. This may be done using an aerosol delivery system such as a pressurized pack or a nebulizer. Nebulizers include air-jet (i.e., pneumatic), ultrasonic, and vibrating-mesh nebulizers, for example with the use of a suitable propellant such as but not limited to dichlorodifluoromethane, trichlorofluoromethane, dichlorotetrafluoroethane, carbon dioxide or other suitable gas. In addition to nebulizers, other devices for pulmonary delivery include but are not limited to metered dose inhalers (MDIs) and dry powder inhalers (DPIs). Capsules and cartridges of for example gelatin for use in an inhaler or insufflator may be formulated containing lyophilized exosomes and a suitable powder base such as lactose or starch.

The EVs, when it is desirable to deliver them systemically, may be formulated for parenteral administration by injection, including for example by bolus injection or continuous infusion. Formulations for injection may be presented in unit dosage form, e.g., in ampoules or in multi-dose containers, with or without an added preservative.

The compositions may take such forms as water-soluble suspensions, solutions or emulsions in oily or aqueous vehicles, and may contain formulation agents such as suspending, stabilizing and/or dispersing agents. Suitable lipophilic solvents or vehicles include fatty oils such as sesame oil, or synthetic fatty acid esters, such as ethyl oleate or triglycerides. Aqueous injection suspensions may contain substances which increase the viscosity of the suspension, such as sodium carboxymethyl cellulose, sorbitol, or dextran. Optionally, the suspension may also contain suitable stabilizers or agents which increase solubility. Alternatively, the exosomes may be in lyophilized or other powder or solid form for constitution with a suitable vehicle, e.g., sterile pyrogen-free water, before use.

The EVs and the composition as described herein will preferably be obtained my means of the process as described above.

The present invention will be now described in more details, referring to examples that are not limitative.

### EXAMPLES AND/OR DESCRIPTION OF FIGURES

### Example 1 - Isolation of the EVs from MSCs

Umbilical cord (UC) derived MSCs, obtained from umbilical cord tissue or Wharton's Jelly was expanded in an expansion unit, comprising a stirred bioreactor (2) in fluid connection with a cell culture medium supply vessel (1). Reference is made to Figure 1, wherein the following numbers refer to:
1. Cell culture medium vessel
2. Stirred bioreactor
3. Cell supernatant vessel
4. Refrigerator
5a, 5b, 5c. Peristaltic pumps
6. First filter unit
7. Second filter unit
8. TFF cassette
9. Final product

The cells are multiplied in the presence of three-dimensional microcarriers present in said bioreactor (2) and in the presence of a xeno-free and serum-free medium comprising purified human albumin at a concentration of 3 g/l. The medium further comprised recombinant purified transferrin at a concentration of 60 mg/l.

The MSCs were allowed to expand and grow until a minimal concentration of at least 40x10⁶ cells/l in the bioreactor (2) was obtained and MSC confluency was maximum 80%. Cell concentration was defined by means of cell counting and fluorescent labelling of MSC adherent to microbeads. At this stage, the cells were ready to start EVs secretion. For this purpose fresh serum-free and xeno-free medium was diluted with basal medium (DMEM/high glucose/no phenol-red/glutamax, Thermo Scientific) 1:10 and supplied to the cell culture. After 24 hrs, the system was ready to start harvesting the EVs. For this purpose, conditioned cell medium or cell supernatant from the bioreactor (2) was pumped to a cell supernatant vessel (3), present in a refrigerator (4) set at 4°C. This supernatant comprised EVs, produced by the MSCs. Supernatant collected in the vessel (3) was subsequently pumped by means of a peristaltic pump (5c) to a first filter unit (6) having a 2 micron filter. The supernatant was filtered by means of dead-end filtration in a closed system with a peristaltic pump (5c) providing constant flow through the filter (6) at 100 mL/min. The flow-through of the first filtration step was subsequently filtered in a second filter unit (7), which was in fluid connection with the first filter unit (6), having a 0.2 micron filter. Filtration was by means of dead-end filtration in a closed system with a peristaltic pump (5c) providing constant flow through the filter (7) at 100 ml/min. Flow through comprising the EVs was collected into a 1l bag, and subsequently stored at 4°C.

In a final step, said flow-through was brought over a TFF cassette (8) having 100 kDa cutoff, which was in fluid connection with the dead-end filter units (6, 7). The retentate of the TFF cassette (8) comprising the EVs was washed with saline buffer solution and concentrated by circulation through the TFF cassette (8) using a peristaltic pump (5c) , which has a rotator speed of 300 rpm, to final volume of 10 mL. The final product (9) was collected from the TFF (8) and either frozen at -80°C or below, or kept at 4°C in cryotubes, bags or other appropriate vessels.

The final product was analyzed to ensure the quality of said product. Quality control included measurement of the concentration of albumin associated with EVs, to ensure the purity and the stability of said product by means of colorimetric analysis and ELISA, particle analysis by means of techniques such as Nanoparticle Tracking Analyzer (NTA), Tunable Resistive Pulse Sensing (TRPS), electron microscopy and/or RAMAN microscopy. A sample of the product was tested for the presence of endotoxins and/or mycoplasma. Other quality controls included qualitative chromatography, mass spectrometry, ELISA, sequencing, qRT-PCR and activity tests such as in vitro T-, B-cells and macrophage polarization.

Under the culturing conditions described above, said MSCs will produce at least 0.25 x 10⁹ EVs/ml cell medium in a time span of 18 to 24 hours of culturing said MSCs.

The experiment was performed with MSCs derived from UCs but has also been repeated by MSCs originating from other tissue or origin, including but not limiting to mammary gland, bone marrow, Wharton's jelly, (cord) blood, peripheral blood, amniotic membrane, adipose tissue, dental pulp, fallopian tube, liver and lung tissue.

### Example 2 - Binding of proteins to EVs

EVs were produced as explained in example 1 up to the collection after the second filtration step. Subsequently, the collected flow through of the filtration steps was transferred to a TFF cassette having a cutoff of 100 kDa for the labeling/binding of EVs with/to annexin V, washing and concentration of the vesicles. All steps were performed in a closed system.

The flow through of the filtration steps was recirculated in a TFF cassette using a peristaltic pump with a rotator speed of 300 rpm, until a volume of 15 ml was obtained. The EVs stayed within the retentate while the permeate was discarded as waste. Subsequently the calcium concentration in the retentate was determined, and calcium was supplemented until a concentration of 2 mM Ca was obtained.

Next, 0.5 mg Annexin V was supplemented to each ml of retentate in the TFF cassette, followed by recirculation of the product for 15 min at room temperature. A small sample was taken to check the annexin V concentration using an ELISA test.

In order to subsequently remove unbound Annexin and unwanted contaminants of < 100 kDa, the retentate (about 15 ml) was washed using a saline washing buffer (about 10 ml), recirculated in the TFF at a reduced speed of 200 rpm, and reconcentrated (200 rpm) to about 15 ml. This washing step was repeated two more times, and the waste (permeate) was checked each time in order to determine the amount of unbound annexin V (ELISA) before being discarded. The third washing permeate contained only very little unbound annexin V, indicating that most of the unbound annexin had been washed away. The peristaltic pump was stopped, and the final concentrated product was subsequently collected in a collection syringe. The product can be aliquoted before storage or directly stored either frozen at -80°C or below, or kept at 4°C in cryotubes, bags or other appropriate vessels.

The experiment has been described for binding of annexin V to EVs, however, it has been repeated in order to obtain EVs bound to thioredoxins and EVs bound to lactadherin.

### Example 3: EVs activity using in vivo models of Crohn's disease

The experiment is demonstrating the activity of EVs associated with Annexin V produced in a similar manner as in example 2 in an in vivo model of Crohn's disease. In short, the experiment is carried out on female mice. Colitis is induced by administration of 3% sodium dodecyl sulfate (DSS) in drinking water, ad libitum administered over five days. This is a well-established and widely used animal model, being referenced hundreds times by Pub Med (for a review, see Kawada et al., 2007). All the animals are treated according to the appropriate ethical codes. Animals are subdivided in three experimental groups.
- Group 1 (normal controls): intraperitoneal (ip) daily injections of PBS (only vehicle), 0,2 ml from 1 to 5 day;
- Group 2 (colitis induction): like group 1, with addition of 3% DSS in drinking water.
- Group 3 (colitis induction and treatment with EVs): like group 2, with addition of MSC-EVs suspended in 0.2 ml PBS by IP route.

On day 6, the animals are sacrificed using CO₂. Colon is excised, and a part of tissue is fixed in formalin for successive histological analysis, whereas another part is immediately frozen in liquid nitrogen and then stored at -80°C for successive RNA extraction.

### Dosage and administration route of EVs

EVs are isolated and administered according to the previously described procedure. Administration route: EVs suspended in 0.2 ml of PBS by intraperitoneal way daily administered from 0 to 5 day.

### Evaluation of intestinal damage

### Animal weight

Disease activity index (faeces evaluation; see Tanaka F, 2008)
- Flogosis histopathological signs
- Titration of inflammation mediator expression in colon tissue extracts: TNFalfa, IL6, IL-1beta, and Cox2 by RT-PCR.

### Statistical analysis of results

Data are expressed as mean ± SD. Group differences are analyzed by t-test p<0.05 being significant.

### Results

In conclusion, the results show that the EVs associated with Annexin V via the process according to the invention are particularly suitable to reduce inflammatory reaction of experimental colitis resulting in reduction of disease activity index and amelioration of general conditions. EVs improve associated with Annexin V the clinical picture and the flogistic response in an animal model of intestinal inflammatory disease.

The present invention is in no way limited to the embodiments described in the examples and/or shown in the figures. On the contrary, methods according to the present invention may be realized in many different ways without departing from the scope of the invention.

## Claims

1. A process for the manufacturing of extracellular vesicles (EVs) associated with proteins, derived from MSCs, said process comprises the steps of:
- purifying EVs from a cell medium comprising MSCs, wherein said purifying occurs via at least one filtration step of said medium; followed by
- a concentration step of the filtrate of said at least one filtration step, wherein said EVs are concentrated by means of tangential flow filtration (TFF) in a TFF device; and
- wherein during said TFF step the EVs are associated with one or more exogenous proteins inside of said TFF device, or in a vessel fluidly connected to said TFF device to which said EVs are transferred from said TFF, said proteins are supplemented to said TFF device or connected vessel during the concentrating of said EVs.

2. Process according to claim 1, wherein calcium is supplemented to said TFF device or connected vessel during concentrating of said EVs.

3. Process according to claim 2, wherein said calcium and said proteins are mixed in said TFF or connected vessel and incubated with said EVs residing in said TFF device or connected vessel.

4. Process according to any of the previous claims, wherein said EVs associated with proteins are washed, re-concentrated, and collected outside said TFF device.

5. Process according to any of the previous claims, wherein said proteins are selected from annexins, thioredoxins and lactadherin.

6. Process according to any of the previous claims, wherein said proteins are Annexin V, Trx or Mfge8, preferably recombinant Annexin V, Trx or Mfge8.

7. Process according to any of the previous claims, wherein said TFF device has a cut-off range of 100 kDa.

8. Process according to any of the previous claims, wherein said MSCs are cultured and expanded in a cell medium comprising purified human serum albumin.

9. Process according to claim 8, wherein the albumin concentration in said medium is between 1 g/l and 5 g/l.

## Patentansprüche

1. Verfahren zur Herstellung extrazellulärer Versikel (EVs), die mit Proteinen assoziiert sind, abgeleitet von MSCs, wobei das Verfahren folgende Schritte umfasst:
- Aufreinigen von EVs aus einem Zellmedium, das MSCs umfasst, wobei das Aufreinigen mittels mindestens eines Schrittes des Filterns des Mediums erfolgt, gefolgt von
- einem Schritt des Konzentrierens des Filtrats aus dem mindestens einen Schritt des Filterns, wobei die EVs mit Hilfe einer Tangentialflussfiltration (TFF) in einer TFF-Vorrichtung konzentriert werden, und
- wobei die EVs während des TFF-Schrittes mit einem oder mehreren exogenen Proteinen im Inneren der TFF-Vorrichtung assoziiert werden oder in einem Gefäß, das in Fluidverbindung mit der TFF-Vorrichtung steht, in das die EVs aus der TFF überführt werden, wobei die Proteine während der Konzentration der EVs in der TFF-Vorrichtung oder dem angeschlossenen Gefäß ergänzt werden.

2. Verfahren nach Anspruch 1, wobei während des Konzentrierens der EVs Calcium in der TFF-Vorrichtung oder dem angeschlossenen Gefäß ergänzt wird.

3. Verfahren nach Anspruch 2, wobei das Calcium und die Proteine in der TFF-Vorrichtung oder dem angeschlossenen Gefäß gemischt und mit den EVs inkubiert werden, die in der TFF-Vorrichtung oder dem angeschlossenen Gefäß vorhanden sind.

4. Verfahren nach einem der vorhergehenden Ansprüche, wobei die EVs, die mit den Proteinen assoziiert sind, gewaschen, erneut konzentriert und außerhalb der TFF-Vorrichtung gesammelt werden.

5. Verfahren nach einem der vorhergehenden Ansprüche, wobei die Proteine aus Annexinen, Thioredoxinen und Lactadherin ausgewählt werden.

6. Verfahren nach einem der vorhergehenden Ansprüche, wobei die Proteine Annexin V, Trx oder Mfge8 sind, vorzugsweise rekombinantes Annexin V, Trx oder Mfge8.

7. Verfahren nach einem der vorhergehenden Ansprüche, wobei die TFF-Vorrichtung einen Abtrennbereich von 100 kDa aufweist.

8. Verfahren nach einem der vorhergehenden Ansprüche, wobei die MSCs in einem Zellmedium kultiviert und expandiert werden, das aufgereinigtes menschliches Serumalbumin umfasst.

9. Verfahren nach Anspruch 8, wobei die Albuminkonzentration in dem Medium zwischen 1 g/l und 5 g/l beträgt.

## Revendications

1. Procédé de fabrication de vésicules extracellulaires (VE) associées à des protéines, dérivées de CSM, ledit procédé comprend les étapes consistant à :
- purifier les VE à partir d'un milieu cellulaire comprenant des CSM, dans lequel ladite purification s'effectue par le biais d'au moins une étape de filtration dudit milieu ; suivi de
- une étape de concentration du filtrat de ladite au moins une étape de filtration, dans lequel les VE sont concentrées au moyen d'une filtration à flux tangentiel (FFT) dans un dispositif FFT ; et
- dans lequel, au cours de ladite étape de FFT, les VE sont associées à une ou plusieurs protéines exogènes à l'intérieur dudit dispositif FFT, ou dans un récipient en communication fluidique avec ledit dispositif FFT dans lequel les VE sont transférées à partir dudit FFT, lesdites protéines étant ajoutées audit dispositif FFT ou récipient communicant pendant la concentration desdites VE.

2. Procédé selon la revendication 1, dans lequel du calcium est ajouté audit dispositif FFT ou récipient communicant pendant la concentration desdites VE.

3. Procédé selon la revendication 2, dans lequel ledit calcium et lesdites protéines sont mélangés dans ledit dispositif FFT ou récipient communicant et incubées avec lesdites VE résidant dans ledit dispositif FFT ou récipient communicant.

4. Procédé selon l'une quelconque des revendications précédentes, dans lequel lesdites VE associées à des protéines sont lavées, reconcentrées et collectées à l'extérieur du dispositif FFT.

5. Procédé selon l'une quelconque des revendications précédentes, dans lequel lesdites protéines sont choisies parmi les annexines, les thiorédoxines et la lactadhérine.

6. Procédé selon l'une quelconque des revendications précédentes, dans lequel lesdites protéines sont l'Annexin V, la Trx ou la Mfge8, de préférence l'Annexin V, la Trx ou la Mfge8 recombinante.

7. Procédé selon l'une quelconque des revendications précédentes, dans lequel ledit dispositif FFT a une plage de coupure de 100 kDa.

8. Procédé selon l'une quelconque des revendications précédentes, dans lequel lesdites CSM sont cultivées et expansées dans un milieu cellulaire comprenant de l'albumine sérique humaine purifiée.

9. Procédé selon la revendication 8, dans lequel la concentration en albumine dans ledit milieu est comprise entre 1 g/l et 5 g/l.
